# EUROPEAN PATENT SPECIFICATION

(11) **EP 0 364 044 B1**
(45) Date of publication and mention of the grant of the patent: **21.09.1994**
(21) Application number: 89202552.9
(22) Date of filing: 09.10.1989
(51) Int. Cl.: A01K 11/00

(54) **Method for implanting an object, and pistol**
Verfahren zum Implantieren eines Objektes und Pistole
Procédé pour implanter un objet, et pistolet

(30) Priority: 10.10.1988 NL 8802482
(43) Date of publication of application: 18.04.1990
(73) Proprietor: TEXAS INSTRUMENTS INCORPORATED, Dallas Texas 75265 (US); Texas Instruments Holland B.V., 7600 AA Almelo (NL)
(72) Inventor: Brouwers, Aroldus Maria, NL-7641 GB Wierden (NL)
(74) Representative: de Bruijn, Leendert C.

(56) References cited:
- WO-A-86/00498
- GB-A- 2 175 186
- GB-A- 2 190 590
- GB-A- 2 217 968
- NL-A- 8 701 027
- NL-A- 8 703 077
- US-A- 3 128 744
- US-A- 3 820 545
- US-A- 4 597 495

## Description

The present invention relates to a method according to the preamble of claim 1.

Such a method is known from US-A-4,597,495, wherein it is described to introduce the object in the ear of the related animal. However, this place of introduction has several drawbacks. It is not unusual that animals (such as pigs) do bite each other (in the ears) or that the wound from the injection of the object irritates the animals. Infection might then occur, which would result in the loss of the transponders.

By having the transponder in the ear of the animal it is relatively easy to fraudulently remove the transponder and replace it. Placing the transponder on a different spot has the considerable drawback that first of all it is not guaranteed that the transponder will not drift in the body and furthermore it is quite difficult during slaughtering to find the transponder back without unacceptable cutting in the meat or within a reasonable time limit. This is quite important especially when glass particles are involved in the transponder. They have to be removed before consumption.

The invention aims to obviate these drawbacks. This is realized according to the characterizing features of the main claim. By having the object such as a transponder injected behind the ear it is not possible that it is removed by biting of other animals. Furthermore it is also not possible that the animal removes the transponder itself by rubbing along a stationary object. After the transponder is introduced it cannot easily be found back and removed off the living animal. However, during slaughtering and more particular during removing of the ear pit it is surprisingly easy to find the transponder back. If an incision is made behind the ear for removing the ear pit, automatically the transponder is found. During practising of the invention it has been found that within a few seconds, five seconds maximum, the transponder is found. Normally the transponder is about 1 cm under the skin of the animal. The place for implantation according to the invention is normally fairly soft so that it is quite easy to inject an object. Drifting does not occur such that the object is readily found back again. Also no problems arise when an animal is growing. If e.g. a transponder is introduced in a pig having a weight of 8 kg and is removed after six months at the time of slaughter when the animal weighs 115 kg it is still easy to find back the transponder.

The invention also relates to a pistol to be used with the method described above according to the preamble of claim 2.

From GB-A-2 190 590 an injector is known having a displaceable abutment. This abutment has an opening to which the needle protrudes and cooperates with a push rod inside the hollow needle to deliver an object in an animal. Such an abutment cannot be used to locate the position near the ear of animal as described above.

This more specifically defined place is achieved according to the characterising features of claim 2. The distance to said point of attachment is accurately defined by holding the stop means, such as a pin, against said attachment point. The implantation angle is also determined by pressing the stop means or a part of the pistol via the stop means against the head of the animal. As the device for making an opening in the skin of the animal comprises a hollow needle, the stop means can also provide protection for the user from injury by this hollow needle and protection from soiling or damaging of this needle while in use.

From WO-A-8600498 it is known to provide a pistol with an abutment means. However, this abutment means is not intended to exactly locate the spot where an object is introduced and cannot be used with the method according to the invention.

The invention will be explained in greater detail below with reference to an example of an embodiment shown in the drawing, in which:
Fig. 1 shows an animal;
Fig. 2 shows a pistol in side view; and
Fig. 3 shows the same pistol in top view.

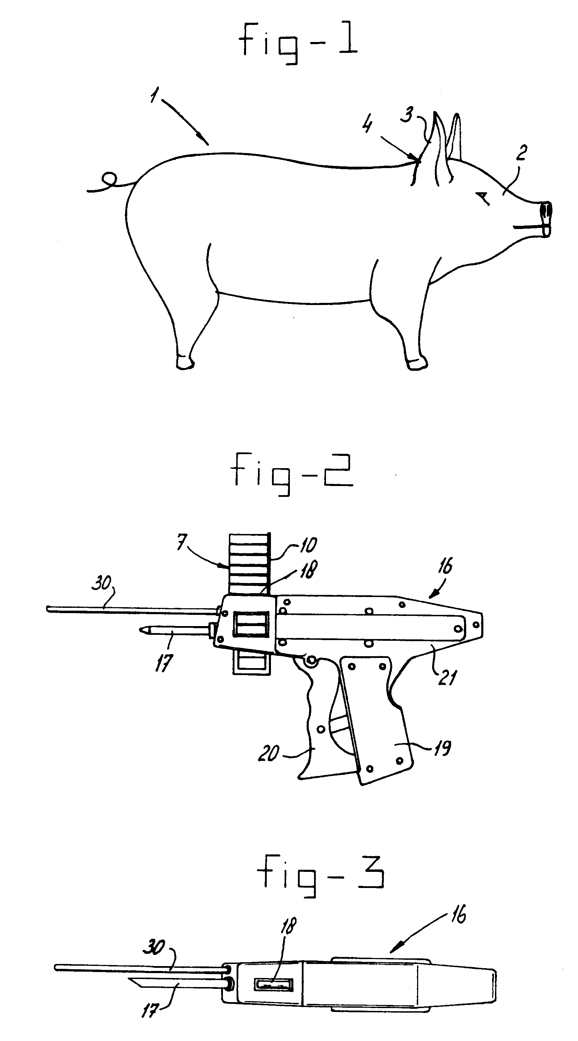

In Fig. 1 an animal, in this case a pig, is indicated by reference number 1. The head of the animal is indicated by 2, while the ear is given reference number 3. According to the invention, an object like a transponder is implanted in the animal immediately behind the attachment point of the ear 3 to the head 2. This implantation point is indicated by 4. This implantation can be achieved with the pistol illustrated in Figs. 2 and 3. This pistol is indicated in its entirety by 16, and comprises a housing 21, trigger 20, grip 19, implantation needle 17 and cartridge holder 18. NL-A series 7 of cartridges 10 is placed in cartridge holder 18 and implanted by injection needle 17 in an opening made previously in the skin of the animal.

A pin 30 is also present, in order to determine the distance of the implantation needle from the attachment point of the ear to the head of the animal. When the implantation is being carried out this pin 30 is guided behind the ear 3 into the corner between ear and head, so that the position of the implantation needle is clearly established both as regards the distance from the attachment of the ear to the head and a regards the angle which the pistol forms with the head. In this way it is ensured that the place where the object is implanted can be found again in a rapid and simple manner at the time of slaughter. It appears that such a pistol is simple to use by anyone, and it is always ensured that the implant position can be found again. Moreover, the presence of stop pin 30 limits the dangerous action of needle 17 to some extent.

## Claims

1. Method for implanting hypodermically in the head of an animal an object with an electronic device which can contain readable information relating to the animal, characterized in that said object is placed in the head of the animal with an injection pistol being positioned at a determined injection location and angle to place said object at a point immediately behind the place where the ear is attached to the head of the animal.

2. A pistol (16) for use in the method according to claim 1, comprising a housing (21) having first and second ends and an intermediate portion; a grip (19) extending from the intermediate portion of said housing; a trigger (20) coupled to said grip extending from the intermediate portion of said housing; a hollow needle (30) protruding from said first end of said housing and having an injection end through which an object can be injected into an animal; locating means protruding from said first end of said housing, characterized in that said locating means comprises a locating pin (17) being on the one hand rigidly connected to the housing and having a free end on the other hand, wherein said free end extends beyond the injection end of the needle.

3. The pistol according to claim 2, wherein said locating pin is offset from said needle.

## Patentansprüche

1. Verfahren, um im Kopf eines Tieres unter der Haut ein Objekt mit einer elektronischen Vorrichtung zu implantieren, die auf das Tier bezogene, lesbare Informationen enthalten kann, dadurch gekennzeichnet, daß das Objekt in den Kopf des Tieres mittels einer Injektionspistole eingebracht wird, die an einer bestimmten Injektionsstelle und in einem bestimmten Injektionswinkel positioniert wird, um das Objekt an einer Stelle unmittelbar hinter dem Ort einzubringen, an dem das Ohr mit dem Kopf des Tieres verbunden ist.

2. Pistole (16) zum Ausführen des Verfahrens nach Anspruch 1, mit einem Gehäuse (21) mit einem ersten und einem zweiten Ende sowie einem Zwischenabschnitt; einem Griff (19), der sich von dem Zwischenabschnitt des Gehäuses erstreckt; einem Abzug (20), der mit dem sich von dem Zwischenabschnitt des Gehäuses erstreckenden Griff verbunden ist; einer Hohlnadel (30), die von einem ersten Ende des Gehäuses hervorsteht und ein Injektionsende aufweist, durch welches ein Objekt in ein Tier injiziert werden kann; sowie einem Lokalisierungsmittel, das von dem ersten Ende des Gehäuses hervorsteht, dadurch gekennzeichnet, daß das Lokalisierungsmittel einen Lokalisierungsstift (17) aufweist, der einerseits starr mit dem Gehäuse verbunden ist und andererseits ein freies Ende aufweist, wobei sich das freie Ende über das Injektionsende der Nadel hinaus erstreckt.

3. Pistole nach Anspruch 2, bei der der Lokalisierungsstift gegen die Nadel versetzt ist.

## Revendications

1. Procédé pour l'implantation hypodermique dans la tête d'un animal d'un objet avec un dispositif électronique qui peut contenir des informations pouvant être lues relatives à l'animal, caractérisé en ce que ledit objet est placé dans la tête de l'animal au moyen d'un pistolet d'injection positionné à un emplacement d'injection et sous un angle déterminés afin de placer ledit objet en un point situé immédiatement à l'arrière de l'endroit où l'oreille est attachée à la tête de l'animal.

2. Pistolet (16) destiné à être utilisé dans le procédé selon la revendication 1, comportant un boîtier (21) ayant des première et deuxième extrémités et une partie intermédiaire, une poignée de saisie (19) s'étendant depuis la partie intermédiaire dudit boîtier ; une gâchette (20) couplée à ladite poignée de saisie s'étendant depuis la partie intermédiaire dudit boîtier ; une aiguille creuse (30) faisant saillie depuis ladite première extrémité dudit boîtier et comportant une extrémité d'injection à travers laquelle un objet peut être injecté dans un animal ; un moyen de positionnement faisant saillie depuis ladite première extrémité dudit boîtier, caractérisé en ce que ledit moyen de positionnement comporte un goujon de positionnement (17) qui est d'une part relié de façon rigide au boîtier et qui comporte d'autre part une extrémité libre qui se prolonge au-delà de l'extrémité d'injection de l'aiguille.

3. Pistolet selon la revendication 2, dans lequel ledit goujon de positionnement est déporté par rapport à ladite aiguille.
